# EUROPEAN PATENT SPECIFICATION

(11) **EP 4 301 440 B1**
(45) Date of publication and mention of the grant of the patent: **06.11.2024**
(21) Application number: 22704788.3
(22) Date of filing: 22.02.2022
(51) Int. Cl.: A61M 15/00, A61M 15/06, A61B 5/091, G01F 1/07, G06M 1/06, G06M 1/12

(54) **HOLDER FOR INHALER ARTICLE WITH INHALATION VOLUME ESTIMATOR**
HALTER FÜR INHALATORARTIKEL MIT INHALATIONSVOLUMENSCHÄTZER
SUPPORT POUR ARTICLE D'INHALATEUR AVEC ESTIMATEUR DE VOLUME D'INHALATION

(30) Priority: 03.03.2021 EP 21160438
(43) Date of publication of application: 10.01.2024
(73) Proprietor: Philip Morris Products S.A., 2000 Neuchâtel (CH)
(72) Inventor: DAYIOGLU, Onur, 2000 Neuchâtel (CH); LANCI, Antonino, 2503 Biel/Bienne (CH); SIGRIST, Martin, 2503 Biel/Bienne (CH); SOTTAS, Loïc, 2503 Biel/Bienne (CH); SPADARO, Fabiana, 2000 Neuchâtel (CH)
(74) Representative: Reddie & Grose LLP
(86) International application number: PCT/EP2022/054429
(87) International publication number: WO 2022/184510

(56) References cited:
- EP-A2- 1 201 258
- WO-A1-2020/178714
- WO-A1-2020/178715
- CH-A5- 627 359
- US-A- 4 292 853
- US-A- 5 158 094
- US-A1- 2015 096 555

## Description

The present disclosure relates to a holder comprising an inhalation volume estimator and to an inhalation system comprising the holder.

In some inhalation systems, it may be beneficial to be able to estimate a volume of an inhalation or a cumulative volume of a plurality of inhalations. For example, some dry powder inhalation systems may comprise a holder and an inhaler article. The inhaler article may comprise a capsule containing a dry powder. In such dry powder inhalation systems, the inhaler article may be engaged with the holder, and the capsule may then be pierced. A user may then inhale on the holder to cause an air flow through the system. Each air flow from each inhalation may carry a portion of the dry powder from the capsule into the lungs of the user. However, after a certain cumulative volume of a plurality of inhalations, subsequent inhalations may not extract a significant amount, if any, of the remaining powder from the capsule. At this point, the capsule may be considered depleted. Thus, by being able to estimate the volume of an inhalation on the system, or the cumulative volume of a plurality of inhalations on the system, it may be possible to indicate when the user has likely finished a capsule.

Some inhalation systems address this need for an inhalation volume estimator using electronic components. However, such electronic components may require complicated user interfaces to operate. In addition, using electronic components can increase the cost and complexity of manufacturing these systems. It is an aim of the present invention to address one or more of these issues.

WO 2020/178714 discloses a holder for an inhaler article including a housing defining an inhaler article cavity. A piercing element is fixed to and extends into the inhaler article cavity. A sleeve is disposed within the inhaler article cavity and is configured to retain an inhaler article. The sleeve is movable along the housing longitudinal axis. A spring element is configured to bias the sleeve toward the open proximal end of the housing.

WO 2020/178715 discloses a piercing device contains a recessed piercing element and is configured to receive a distal end of an inhaler article. The piercing element pierces or punctures a single hole into a capsule contained within the inhaler article when the inhaler article is seated into an inhaler article cavity of the piercing device. The piercing device includes a marking element extending into the inhaler article cavity from the housing inner surface. The marking element is configured to mark an outer surface of an inhaler device when the inhaler device is received within the inhaler article cavity. The marking element extends orthogonally to the piercing element longitudinal axis.

US 5158094 discloses an incentive spirometer that indicates the volume of air drawn therethrough. The spirometer comprises a housing with a turbine rotatably mounted therein. The turbine is provided with a take-off shaft with which is operably associated a signal device responsive to the rotation of the shaft. Air drawn through the spirometer by a patient rotates the turbine and the signal device indicates the volume of air that has passed through the spirometer.

US 4292853 discloses a respiratory air or gas flow volume indicating instrument comprising a tubular stator within a casing which forms an annular gallery around the stator. Oblique slots are formed through the stator so that air or gas flow through them forms a spirally swirling flow which drives a rotor. A barrier constrains air or gas flow in one direction round the gallery. The length of each slot is increased progressively in the direction of flow around the stator from a radial port to the barrier. The slots open at one end of the stator and have sloping side and end faces so that the aperture at the radially inner end of each slot is smaller than the aperture at the radially outer end of that slot.

CH 627359 discloses a spirometer which improves measurement accuracy by direct conversion of a flow rate into a pulse sequence, immediate counting of which immediately results in the required value after multiplication by a constant factor. It is especially advantageous to use a stepping motor on a shaft of which an impeller wheel lying in the respiratory flow is mounted. At the same time, a multiplicity of exactly defined pulses is obtained for each revolution of the shaft. The result of counting and multiplication is displayed digitally.

US 2015/096555 discloses an administration method for a medical spray inhaler and the medical spray inhaler, which utilizes an inhaling sensing unit to sense an air flow change for generating an inhaling signal after a user inhales by an inhaler body. A control unit is then used to receive the inhaling signal and calculate a delay time. After the delay time, the control unit outputs a control instruction to control a medicine container disposed on the inhaler body to spray a medical spray and concurrently records the ventilation volume per inhalation of the user.

EP 1201258 discloses a process for optimizing dose precipitation in an inhalation unit for the application of medication. The process comprises determining breathing volume in order to optimize dose. The medication particle concentration is then matched to the volume, in the form of an aerosol. A signal is produced once the volume has been reached.The invention is defined in the appended independent claim, to which reference should now be made. Optional features of the invention are defined in dependent claims. Aspects, embodiments or examples falling outside the scope of the appended independent claim are not part of the invention, and are merely included for illustrative or explanatory purposes.

According to a first aspect of the disclosure, there is provided a holder according to claim 1.

Advantageously, the indicator may be able to indicate useful information to the user. For example, the indicator may be able to allow a user to determine that a consumable, such as a consumable of an inhaler article, has been fully consumed.

The holder may comprise, or may be, an aerosol-generating device. The holder and the inhaler article may together be considered an inhalation system. In use, a user may inhale on the inhalation system, for example on the inhaler article or on a mouthpiece of the holder. Such an inhalation may result in the air flow through the air flow passage. References herein to inhalations and air flows through the air flow passage may therefore be used interchangeably.

The turbine may be at least partially located, or locatable, in the air flow passage. The turbine may comprise a plurality of blades. One or more blades of the turbine may be at least partially located, or locatable, in the air flow passage. In use, air flow through the air flow passage may contact the turbine, for example one or more blades of the turbine. This may cause the turbine to rotate.

The indicator may comprise an indicator component. The indicator or indicator component may be coupled, for example indirectly coupled by a series of gears, to the turbine. The indicator may comprise a reference component. The indicator component may be configured to move, for example one or both of rotate and translate, relative to the reference component as the turbine rotates. The indicator component may comprise a pointer. The indicator component may comprise a scale. The indicator component may comprise an indication surface. The reference component may comprise a reference pointer. The reference component may comprise a reference scale. The reference component may comprise a reference window.

As an example, the indicator component may comprise a pointer and the reference component may comprise a scale. And the pointer may be coupled to the turbine and configured to move relative to the scale as the turbine rotates.

Advantageously, relative movement between an indicator component and a reference component may provide a reliable and straightforward way to indicate the inhalation volume information to the user.

The indicator may be coupled to the turbine so as to rotate when the turbine rotates. For example, the indicator component of the indicator may be coupled to the turbine so as to rotate when the turbine rotates. Notably, reference to rotation of the indicator may refer to rotation of a portion of the indicator or to rotation the indicator component of the indicator. References to rotation of the indicator do not necessarily refer to rotation of all of the indicator.

Advantageously, this may allow the use of a straightforward and reliable coupling between the indicator and the turbine so that rotation of the turbine alters the inhalation volume information indicated by the indicator.

The inhalation volume estimator may be configured such that a rate of rotation of the indicator or indicator component is less than a rate of rotation of the turbine. For example, the inhalation volume estimator may be configured such that, if the indicator or indicator component were to undergo a complete 360 degree rotation of the indicator, the turbine would undergo at least 10, 20, 30, 50 or 70 complete 360 degree rotations. For the avoidance of doubt, neither this passage, nor any other passage referring to a 360 degree rotation of the indicator or indicator component, is intended to imply that the indicator or indicator component is necessarily able to undergo a complete 360 degree rotation. Such passages may refer simply to a rate of rotation of the indicator or indicator component, regardless of whether the degree of rotation is limited.

For each 360 degree rotation of the turbine, the indicator or indicator component may be configured to rotate by less than 36, 24, 18, 12, 10, 8, or 5 degrees.

Advantageously, this may allow the use of a turbine which spins relatively freely and therefore offers relatively low resistance to air flow through the air flow passage.

The holder or inhalation volume estimator may be configured to prevent, resist, or minimise air flow through the air flow passage in a reverse direction. As used herein, the term "reverse direction" may refer to a direction of air flow which is opposite to a direction of air flow when a user inhales on the inhalation system.

The turbine may be able to rotate in a first direction. The turbine may be unable to rotate in a second direction opposite to the first direction. The turbine may resist rotation in a second direction more than rotation in a first direction opposite to the second direction. For example, the inhalation volume estimator may comprise one or more of a ratchet and a freewheel clutch. One or both of the ratchet and the freewheel clutch may be coupled to the turbine. One or both of the ratchet and the freewheel clutch may be coupled to the turbine so as to prevent or resist rotation of the turbine in the second direction opposite to the first direction.

Advantageously, this may prevent or discourage air flow through the air flow passage in a reverse direction. This may prevent air flow through the air flow passage in a reverse direction from altering the inhalation volume information. In addition, where a source of an aerosol component such as a dry powder is located downstream of the turbine, preventing air flow in a reverse direction may reduce a risk of the aerosol component clogging the turbine.

Alternatively, or in addition, the indicator may be configured such that air flow through the air flow passage in a reverse direction cannot alter the inhalation volume information. For example, the indicator may be configured such that rotation of the turbine in a direction corresponding to a reverse direction of air flow cannot alter the inhalation volume information.

The indicator, for example the indicator component of the indicator, may be coupled to the turbine by one or more gears. The indicator or indicator component may be coupled to the turbine, for example by one or more gears, such that a rotary transmission ratio between the indicator or indicator component and the turbine is at least 1:10, 1:20, 1:30, 1:50 or 1:70. As such, for each complete 360 degree rotation of the indicator or indicator component, the turbine may undergo at least 10, 20, 30, 50 or 70 complete 360 degree rotations.

Advantageously, this allows the use of a turbine which spins relatively freely and therefore offers relatively low resistance to air flow through the air flow passage. In addition, the use of one or more gears may provide a long-lasting and reliable coupling between the indicator and the turbine.

The indicator or indicator component may be able to rotate at least 30, 60, 90, 180 or 360 degrees in a given direction. Advantageously, a larger possible degree of rotation may allow a user to more precisely assess the information indicated by the indicator.

The indicator or indicator component may be able to rotate indefinitely in a given direction. Advantageously, this may allow resetting of the indicator without needing the indicator to be able to rotate in both directions.

The indicator or indicator component may be able to rotate less than 60, 90, 180 or 360 degrees in a given direction. Rotation of the indicator or indicator component may be limited between two angular positions separated by an angle of less then 60, 90, 180 or 360 degrees.

Alternatively, or additionally, to rotating, the indicator or indicator component may be configured to translate, for example translate linearly, as the turbine rotates.

The inhalation volume information relating to a volume of an air flow through the air flow passage may be, or may comprise, information relating to a cumulative volume of a plurality of temporally separated air flows through the air flow passage. Thus, the indicator may be suitable for indicating, or configured to indicate, inhalation volume information relating to a cumulative volume of a plurality of temporally separated air flows through the air flow passage. For example, the indicator may be suitable for indicating, or configured to indicate, inhalation volume information relating to a cumulative volume of all air flows through the air flow passage since the indicator was last reset.

Advantageously, this may allow the indicator to provide more useful inhalation volume information to a user. This is because this may allow the indicator to provide information relating to several of the user's previous inhalations.

The inhalation volume information may be, or may comprise, an indication of a volume of an air flow through the air flow passage. The inhalation volume information may be, or may comprise, an indication of a cumulative volume of a plurality of temporally separated air flows through the air flow passage. As used herein, the term "temporally separated air flows" refers to air flows that are separated in time. Thus, the inhalation volume information may be, or may comprise, an indication of a cumulative volume of a plurality distinct air flows, for example air flows corresponding to distinct inhalations on the inhalation system.

As an illustrative example, the indicator component may comprise a coloured surface and the reference component may comprise a window. In use, in response to inhalations on the inhalation system (resulting in corresponding air flows through the air flow passage), the coloured surface may move relative to the window so as to gradually fill the window. In this sense, the indicator may be considered a form of progress bar. Thus, the inhalation volume information may be presented in the form of a progress bar. The progress bar may initially indicate zero progress. This may inform the user that a cumulative volume of air flow through the air flow passage is zero since the indicator was last reset. The progress bar may, in response to one or more inhalations on the inhalation system (resulting in one or more corresponding air flows through the air flow passage), indicate further progress along the progress bar. In this illustrative example, this further progress may be indicated by the coloured surface moving further so as to fill more of the window. If the indication means is configured for use with a particular consumable usually requiring a cumulative volume of X m³ to fully consume, and the progress bar indicates 50 percent progress after six inhalations, this may allow the user to estimate that around six inhalations of a similar volume are remaining before the consumable has been fully consumed. After X m³ of air has flowed through the air flow passage, the progress bar may indicate 100 percent progress. In this illustrative example, this may be indicated by the coloured surface completely filing the window. This may indicate to the user that the consumable has likely been fully consumed.

The inhalation volume information may be, or may comprise, an indication of a remaining volume of air flow through the air flow passage before the cumulative volume of a plurality of air flows through the air flow passage reaches a threshold, for example the threshold at which a consumable of the inhaler article has likely been fully depleted.

The indicator, for example the reference component of the indicator, may comprise one or more targets. The one or more target may correspond to a desirable volume of an inhalation or a desirable volume of an air flow through the air flow passage. The one or more targets may correspond to a desirable cumulative volume of a plurality of inhalations or a desirable cumulative volume of a plurality of air flows through the air flow passage. Advantageously, this may allow a user to determine whether their inhalations are sufficiently deep, or high-volume, after relatively few inhalations.

For example, where the indicator is in the form of a progress bar, and the inhaler article is intended to be consumed in twelve inhalations, the indicator may comprise targets, or markings, dividing the progress bar into twelve equal portions. Thus, after each inhalation, a user may be able to determine whether the volume of their inhalation (resulting in a corresponding volume of air flow through the air flow passage) was smaller or larger than desirable. This may be particularly advantageous where inhalation systems require deep, or high-volume, inhalations to maximise effectiveness.

The inhalation volume estimator may comprise a secondary indicator configured to alert the user at an alert point. The secondary indicator may be configured to alert the user with one or both of tactile and audible feedback.

The inhalation volume estimator may comprise a tertiary indicator configured to alert the user at the alert point or at a second alert point. The tertiary indicator may be configured to alert the user with one or both of tactile and audible feedback.

Advantageously, tactile or audible feedback may alert the user without the user having to perform a visual check of a visual indicator.

As a user inhales on the inhalation system, the alert point may occur before the second alert point.

The alert point may correspond to a cumulative volume of air flow through the air flow passage reaching a predetermined volume. The second alert point may correspond to a cumulative volume of air flow through the air flow passage reaching a second predetermined volume. The predetermined volume and the second predetermined volume may be the same or different. One or both of the predetermined volume and the second predetermined volume may be an estimate of the volume required for a consumable, such as an inhaler article, to be fully consumed.

Advantageously, one or both of the secondary indicator and the tertiary indicator may alert the user when the consumable has likely been fully consumed. Advantageously, where the alert point and the second alert point occur at different times, for example where the predetermined volume and the second predetermined volume are different, the user may be provided with a warning prior to a point at which the consumable has likely been fully consumed.

As an illustrative example, a secondary indicator may be used to provide audible or tactile feedback, such as a clicking noise, when a consumable of an inhaler article is likely almost fully consumed, for example likely around 80 or 90% consumed. Subsequently, the tertiary indicator may provide audible or tactile feedback, such as an increase in resistance to draw on the inhalation system, when a consumable of an inhaler article is likely fully consumed.

The inhalation volume estimator may comprise a live flow rate indicator. The live flow rate indicator may allow a user to determine the current rate of air flow through the air flow passage, for example, as the user inhales on the inhaler article. The live flow rate indicator may comprise an fixed scale showing an optimum air flow rate allowing a user to advantageously determine whether their inhalation is at the optimum air flow rate. The optimum airflow rate may be configured to provide optimum delivery of dry powder from the capsule of the inhaler article, or may be selected to provide an optimum duration of user experience from a single inhaler article. In use, a user may adjust their inhalation to match the optimum air flow rate as shown by the live flow rate indicator.

The live flow rate indicator may comprise a fixed scale showing an upper optimum air flow rate limit and a lower optimum air flow rate limit. In this way, a user may try to keep their inhalation flow rate between the two optimum air flow rate limits in use.

The live flow rate indicator may determine the live flow rate based on the speed of the rotation of the turbine. The live flow rate indicator may comprise a float, ball, or rotameter type flowmeter.

The inhalation volume estimator may comprise a stop. One or both of the secondary indicator and the tertiary indicator may comprise the stop. The stop may limit motion, for example rotation, of the indicator or indicator component in a given direction. The indicator, or indicator component, may be configured to move, for example rotate, as the turbine rotates until the indicator or indicator component contacts the stop. The stop may be configured to prevent rotation, or increase the resistance to rotation, of the turbine in a given direction, for example at the alert point or the second alert point, which may be when the indicator or indicator component contacts the stop. The stop may provide feedback to the user by increasing a resistance to draw of the air flow passage. This may be due to rotation of the turbine being prevented or resisted.

Advantageously, the stop may prevent further rotation in a first direction. Advantageously, the stop may provide a reliable secondary indicator.

The inhalation volume estimator may comprise a second stop. The second stop may limit motion, for example rotation, of the indicator or indicator component in a direction different, for example opposite, to the direction of motion which is limited by the stop described above.

Advantageously, the combination of a stop and a second stop may limit motion, for example rotation, of the indicator or indicator component between two limits, for example between two angular positions.

The turbine may be an axial inflow turbine. The turbine may be a radial inflow turbine.

The inhalation volume estimator may be a mechanical inhalation volume estimator. The inhalation volume estimator may consist of non-electrical components. The inhalation volume estimator may function absent an electrical power source. Advantageously, this may make the inhalation volume estimator cheaper to manufacture.

The inhalation volume estimator comprises a modifying mechanism. The modifying mechanism is coupled to the indicator, for example to one or both of the indicator component and the reference component of the indicator. The modifying mechanism is for resetting or otherwise modifying the inhalation volume information. The modifying mechanism may be configured to allow the user to set the inhalation volume information. One or more of the indicator, the indicator component and the reference component may be moveable so as to to reset or otherwise modify the inhalation volume information. For example, the inhalation volume estimator may be configured such that a user is able to physically move one or more of the indicator, the indicator component and the reference component to reset or otherwise modify the inhalation volume information, for example using their hands or a suitable tool.

Advantageously, allowing the user to set the inhalation volume information may allow the user to tailor their experience. For example, a user may wish to ensure that each inhalation is a certain volume, and allowing the user to set the inhalation volume information may allow the user to achieve this.

The holder and inhaler article may together be considered an inhalation system.

In use, the holder may be configured to generate or introduce an aerosol. The holder may be configured to generate or introduce an aerosol downstream of the turbine. The holder may be configured to generate or introduce an aerosol in the air flow passage or downstream of the air flow passage. Advantageously, generating or introducing the aerosol downstream of the turbine may reduce a likelihood of an aerosol component of the aerosol clogging or otherwise negatively impacting the function of the turbine.

The holder may comprise an air flow inlet. The holder may comprise an air flow outlet. An air flow passage, such as the air flow passage referred to in relation to the first aspect, may extend between the air flow inlet and the air flow outlet. The holder may comprise a mouthpiece.

The holder may be configured to engage with the inhaler article. The holder may be configured to receive at least a portion of the inhaler article. The holder may comprise a cavity for receiving at least a portion of the inhaler article. The holder may comprise a housing. The housing may define the cavity. The housing may define one or both of the air flow inlet and the air flow outlet. At least a portion of the indicator, for example the reference component of the indicator, may be coupled to, for example fixed to, the housing of the holder.

In use, a user may inhale on an inhaler article engaged with the holder, or on a mouthpiece of the holder. This may result in an air flow in through the air flow inlet, then through the air flow passage, then out through the air flow outlet. The air flow may then flow through or past the inhaler article. Air flowing through or past the inhaler article may entrain an aerosol component from the inhaler article. This may form an aerosol. The aerosol component may comprise one or more of a solid aerosol component such as a dry powder, a liquid aerosol component such as a suspended droplet of a liquid, and a gaseous aerosol component such as a vaporised liquid aerosol component. The air flow may entrain a solid aerosol component or a liquid aerosol component to form an aerosol. The air flow may entrain a gaseous aerosol component which subsequently cools and condenses to form an aerosol. The aerosol may then flow into the mouth of the user, for example through the inhaler article or the mouthpiece.

The holder may comprise an activation means, for example a piercing or rupturing means. The piercing or rupturing means may comprise a piercing element such as a spike. The piercing or rupturing means may be configured to pierce or rupture the capsule of the inhaler article. The piercing or rupturing means may be configured to extend into the cavity to pierce or rupture the capsule of the inhaler article. The holder may comprise a sleeve. The sleeve may be moveable relative to the piercing or rupturing means. The sleeve may define the cavity. In use, an inhaler article may be partially received in the cavity defined by the sleeve. The sleeve and inhaler article may be configured to move together relative to a housing of the holder. The sleeve and inhaler article may be configured to move together relative to the piercing or rupturing means, for example until the piercing or rupturing means pierces or ruptures a capsule of the inhaler article.

Movement of the sleeve relative to a housing of the holder may be used to operate the modifying mechanism.

As used herein, operation of the modifying mechanism may refer to resetting or otherwise modifying the inhalation volume information.

The inhaler article may comprise or may be a consumable, for example the consumable referred to in relation to the first aspect. The inhaler article may comprise a source of an aerosol component, for example a dry powder or an aerosol-forming substrate. The inhaler article may comprise a capsule, for example a capsule containing a dry powder. The holder may comprise or may be a dry powder inhaler.

An illustrative example of use of an exemplary holder is explained below.

In use, a user may engage a holder with an inhaler article comprising a capsule containing a dry powder. The user may then operate the holder so as to pierce the capsule with a piercing element of the holder. The user may then inhale on the inhaler article, or on a mouthpiece of the holder. Such an inhalation may result in air flowing in through the air flow inlet of the holder. This air may then flow through the air flow passage. The air flow may alter the inhalation volume information indicated to the user by the indicator of the inhalation volume estimator. The air flow through the air flow passage may entrain the dry powder from the capsule of the inhaler article. The air and dry powder may form an aerosol in the air flow passage or downstream of the air flow passage. The aerosol may then be delivered to the user. After the consumable has been fully consumed, the inhaler article may be disengaged from the holder. The holder may be then be usable with another, fresh inhaler article.

The modifying mechanism is operable by one or both of engaging the inhaler article with the holder and disengaging the inhaler article from the holder. The modifying mechanism may be configured to reset the indicator (or equally reset the inhalation volume information) in response to one or both of engaging the inhaler article with the holder and disengaging the inhaler article from the holder.

Advantageously, this may avoid the need for a separate action to reset the indicator.

As used herein, and in particular with reference to the modifying mechanism, engaging the inhaler article with the holder may comprise one or both of receiving at least a portion of the inhaler article in the holder, and motion of the inhaler article relative to the holder. The motion of the inhaler article relative to the holder may occur when at least a portion of the inhaler article is received in the holder. The motion of the inhaler article relative to the holder may comprise motion to activate or pierce or rupture a source of aerosol component such as a capsule of the inhaler article.

As used herein, and in particular with reference to the modifying mechanism, disengaging the inhaler article from the holder may comprise one or both of withdrawal of at least a portion of the inhaler article from the holder, and motion of the inhaler article relative to the holder. The motion of the inhaler article relative to the holder may occur when at least a portion of the inhaler article is received in the holder. The motion of the inhaler article relative to the holder may comprise motion of the inhaler article following activation, piercing, or rupturing a source of aerosol component such as a capsule of the inhaler article.

The holder may comprise an inhalation counter for counting a number of inhalations on the holder. The inhalation counter may be an electronic inhalation counter.

The holder may comprise an inhalation count indicator for indicating inhalation count information relating to an inhalation count of the inhalation counter.

Advantageously, the inhalation count indicator may indicate useful information to a user. Advantageously, the combination of an inhalation counter and an inhalation volume estimator may give a user more information about their inhalations. This may allow the user to more accurately determine when a consumable has been fully consumed.

The holder may be a mechanical holder. The holder may consist of non-electrical components. The holder may function absent an electrical power source. Advantageously, this may make the holder cheaper to manufacture.

According to a second aspect of the disclosure, there is provided an inhalation system. The system comprises a holder according to the first aspect and an inhaler article comprising a source of an aerosol component.

The inhaler article may comprise a dry powder. The source of the aerosol component may comprise or be a capsule. The capsule may comprise a dry powder.

The capsule may contain one or both of nicotine particles and flavour particles. The flavour particles may be larger than the nicotine particles. In use, the flavour particles may assist in transporting the nicotine particles into the lungs of the user. The flavour particles may preferentially remain in the mouth or buccal cavity of the user. In use, one or both of nicotine particles and the flavour particles may be delivered at flow rates that are within conventional smoking regime flow rates.

As used herein, the term "nicotine" may refer to nicotine and nicotine derivatives such as free-base nicotine, nicotine salts and the like.

As used herein, the term "flavour" may refer to organoleptic compounds, compositions, or materials that alter and are intended to alter the taste or aroma characteristics of nicotine during consumption or inhalation thereof.

Examples will now be further described with reference to the figures in which:
Figure 1 is a cross-sectional view of an inhalation system comprising a holder and an inhaler article;
Figure 2 is a cross-sectional view of an inhalation volume estimator of the inhalation system of Figure 1; and
Figure 3 is a cross-sectional view of an alternative inhalation volume estimator.

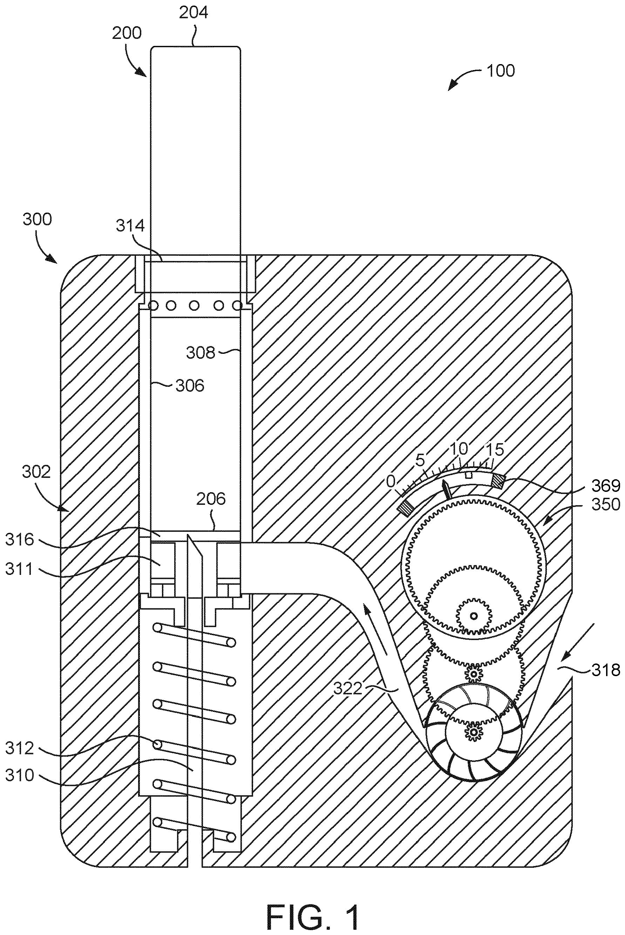

Figure 1 is a cross-sectional view of an inhalation system 100. The inhalation system 100 comprises an inhaler article 200 and a holder 300 receiving the inhaler article 200.

The inhaler article 200 includes a body extending along an inhaler longitudinal axis from a mouth end 204 to a distal end 206. The mouth end 204 is for insertion into a mouth of a user. The distal end 206 opposes the mouth end 204. The inhaler article 200 comprises a capsule cavity disposed within the body and bounded downstream by a filter element and bounded upstream by an open tubular element defining a central passage. As used herein, the terms "upstream" and "downstream" refer to relative positions of components relative to a direction of air flow through the system during normal use. The inhaler article 200 comprises a capsule disposed within the capsule cavity. The central passage of the open tubular element forms an air inlet aperture extending from the distal end of the body to the capsule cavity. The central passage has a smaller diameter than the capsule. As such, the capsule cannot fall through the central passage. The capsule comprises a dry powder. The dry powder comprises nicotine particles and flavour particles. The capsule comprising the dry powder may be referred to as a consumable of the inhaler article 200.

The holder 300 comprises a housing 302 defining a housing cavity. The holder 300 comprises a movable sleeve 306. The housing cavity is for receiving and retaining the sleeve 306. The sleeve 306 defines a sleeve cavity 308. The sleeve cavity 308 is for receiving and retaining the inhaler article 200. In Figure 1, the inhaler article 200 is shown received in the sleeve cavity 308, and the sleeve 306 is shown received in the housing cavity.

The holder 300 comprises a piercing element 310 fixed to a distal end of the housing 302 and extending along a longitudinal axis of the holder 300 towards the housing cavity. The piercing element 310 is configured to activate or pierce the capsule disposed within the inhaler article 200. The holder 300 comprises a spring 312 configured to bias the sleeve 306 away from the piercing element 310. The holder comprises a sleeve resting component 311. In a resting position of the holder 300, as shown in Figure 1, the sleeve 306 rests on the sleeve resting component 311 and the sleeve resting component 311 rests on the spring 312. The sleeve resting component 311 comprises a central opening. In use, the piercing element 310 is able to extend through the central opening of the sleeve resting component 311 when the sleeve 306 and sleeve resting component 311 are moved towards the piercing element 310 against the action of the spring 312.

The sleeve 306 comprises a first open end 314 and a second opposing end 316. The second opposing end 316 of the sleeve 306 comprises a central opening. This central opening is configured to allow an air flow to enter the sleeve cavity 308. This central opening is configured to allow the piercing element 310 to enter the sleeve cavity 308 when the sleeve 306 is moved along the longitudinal axis of the holder 300 towards the piercing element 310.

The holder 300 comprises an air flow inlet 318 and an air flow passage 322 extending from the air flow inlet 318 to the second end 316 of the sleeve 306. The holder 300 comprises an inhalation volume estimator 350 coupled to the air flow passage 322.

In use, a user inserts the inhaler article 200 into the sleeve cavity 308 of the holder 300. The inhalation system 100 appears as shown in Figure 1 at this stage. The user then presses the inhaler article 200 in the direction of the distal end 206 of the inhaler article 200. This compresses the spring 312 and moves the inhaler article 200, the sleeve 306, and the sleeve resting component 311 in a distal direction relative to the housing 302. As this happens, the piercing element 310 extends through the central opening of the sleeve resting component 311, then through the central opening of the sleeve 306, then into the sleeve cavity 308, then into the capsule cavity, before contacting and piercing the capsule. The user may then release the inhaler article 200. This will allow the spring 312 to expand and move the inhaler article 200, the sleeve 306, and the sleeve resting component 311 in a proximal direction relative to the housing 302, back to the resting position shown in Figure 1. However, at this stage, the capsule has been pierced.

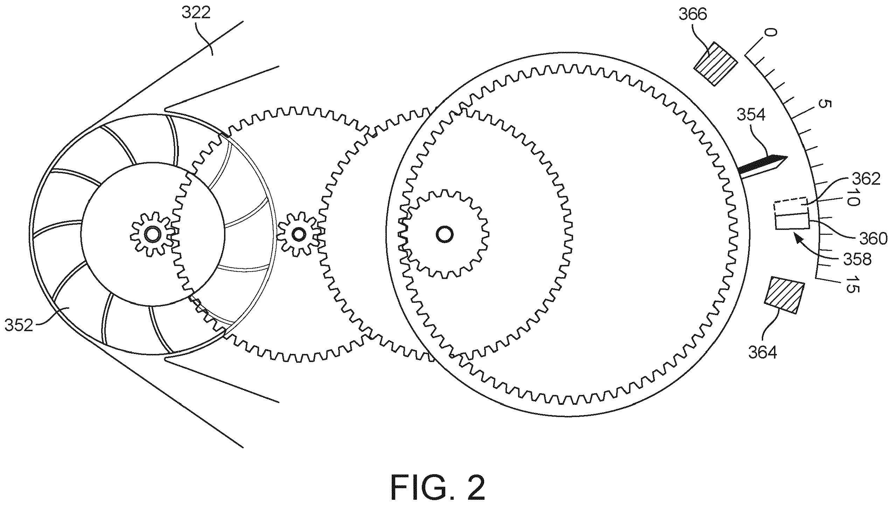

A user may then inhale on the mouth end 204 of the inhaler article 200. This causes air to flow in through the air flow inlet 318 of the holder 300 and through the air flow passage 322. This air flows through the central opening of the sleeve 306, then through the central opening of the inhaler article 200 and into the capsule cavity. Dry powder from the capsule is entrained by this air flow to form an aerosol. This aerosol flows through the filter element and is delivered to the user. The air flow passage is indicated with several arrows in Figure 1. The user may repeatedly inhale on the inhaler article 200. Each inhalation results in a corresponding air flow through the air flow passage 322. This air flow interacts with the inhalation volume estimator 350 so as to alter inhalation volume information indicated by an indicator of the inhalation volume estimator 350. The interaction between the air flow and the inhalation volume estimator 350 is explained in more detail below with reference to Figure 2. After the capsule is likely fully consumed, for example after a cumulative volume of a plurality of air flows through the air flow passage 322 of around fifteen litres, the indicator indicates this to the user. The user may then withdraw the inhaler article 200 from the holder 300 and dispose of it.

Figure 2 shows the inhalation volume estimator 350 of the holder 300 shown in Figure 1. The inhalation volume estimator 350 comprises a radial inflow turbine 352 and an indicator coupled to the turbine 352 by a series of gears for indicating inhalation volume information relating to a volume of an air flow through the air flow passage 322. In the embodiment shown in Figures 1 and 2, the inhalation volume information is an indication of the cumulative volume of all air flows through the air flow passage 322 since the indicator was last reset.

The indicator comprises an indicator component 354 in the form of a pointer and a reference component in the form of a scale on the housing 302 of the holder 300. The indicator component 354 is visible through a window in the housing 302 of the holder 300. The turbine 352 is configured to rotate in response to air flow through the air flow passage 322, thereby rotating the indicator component 354 and altering the inhalation volume information indicated by the indicator.

The indicator component 354 is coupled to the turbine 352 by a series of gears. The indicator component 354 is configured to rotate when the turbine 352 rotates. However, the rate of rotation of the indicator component is significantly less than the rate of rotation of the turbine 352.

The indicator component 354 is configured to move relative to the reference component as the turbine 352 rotates so as to alter the inhalation volume information indicated to the user. The reference component, in the form of a scale, comprises markings numbered from 0 to 15. In the embodiment shown in Figures 1 and 2, these markings correspond to a cumulative volume of all air flows through the air flow passage 322 since the indicator was last reset to 0. As such, for a litre of air flow through the air flow passage 322 (for example resulting from an inhalation on the inhalation system 100 which is approximately 1 litre in volume), the indicator component 354 rotates clockwise relative to the reference component so as to point at a number on the reference component which is 1 greater than the number which the indicator component 354 was pointing at prior to the litre of air flow through the air flow passage 322. So, as an example, if the indicator has just been reset to 0 and a user inhales on the inhalation system 100 so as to cause 2 litres of air to flow through the air flow passage 322, the indicator component 354 will rotate from 0 to 2. If the user inhales on the system 100 again but this time the inhalation results in 1 liter of air flow through the air flow passage 322, the indicator component 354 will rotate from 2 to 3.

In other embodiments, the indicator may indicate the inhalation volume information in other ways. For example, the indicator may comprise one or more of a linear scale, a rotary scale, and an electronic display. And the inhalation volume may comprise one or both of an indication of a cumulative volume of a plurality of temporally separated air flows through the air flow passage (such as in the embodiment of Figures 1 and 2), and an indication of a remaining volume of air flow through the air flow passage before the cumulative volume of air flow through the air flow passage reaches a threshold, for example the threshold at which a consumable of the inhaler article has likely been fully depleted.

The inhalation volume estimator 350 comprises a secondary indicator 358 configured to alert the user at an alert point with audible feedback. The secondary indicator 358 comprises a flexible cantilever 360 and a striking surface 362. The cantilever 360 comprises a free end and a fixed end. The fixed end is fixed to the housing 302 of the holder 300. The indicator component 354 is configured to contact the free end of the cantilever 360 as the indicator component 354 rotates. Specifically, the indicator component 354 contacts the free end of the cantilever 360 once the indicator component 354 reaches around 11 on the reference component. The indicator component 354 contacting the free end of the cantilever 360 causes the cantilever to flex, or bend, about the fixed end as the indicator component 354 rotates further, between 11 and 12 on the reference component. Eventually, the indicator component 354 rotates sufficiently far so as to disengage from the free end of the cantilever 360. This occurs once the indicator component 354 reaches around 12 on the reference component. This releases the cantilever 360 and allows the cantilever 360 to spring back to its original position under its own resilience. This causes a surface of the cantilever 360 to strike the striking surface 362, thereby generating a clicking noise. Thus, the alert point corresponds to a cumulative volume of air flow through the air flow passage 322 reaching a predetermined volume of 12 litres, and the user is provided with audible feedback at the alert point. In this case, the alert point lets the user know that approximately 80% (12/15) of the consumable of the inhaler article 200 has likely been consumed.

With reference to Figure 2, the cantilever 360 extends further in the direction of the page than the indicator component 354, and the striking surface 362 sits below the plane in which the indicator component 354 rotates. As such, the indicator component 354 rotates above and past the striking surface 362 to contact an upper portion of the cantilever 360, and when the cantilever 360 returns to its original position, a lower portion of the cantilever 360 strikes the striking surface 362.

The inhalation volume estimator 350 comprises a tertiary indicator 364 configured to alert the user at a second alert point. The tertiary indicator 364 is in the form of a stop fixed to the housing 302 of the holder 300 and is configured to alert the user with tactile feedback at a second alert point. As explained above, the indicator component 354 rotates towards 15 on the reference component as the user inhales on the inhalation system 100. Once the indicator component 354 reaches 15 on the reference component, the indicator component 354 contacts the secondary indicator 358. This prevents further rotation of the indicator component 352. Since the indicator component 354 is coupled to the turbine 352, preventing further rotation of the indicator component 354 also prevents further rotation of the turbine 352. Thus, with the turbine 352 being unable to rotate and partially blocking the air flow passage 322, a resistance to draw of the air flow passage 322 increases. The user will notice this increase in resistance to draw as they attempt to inhale on the inhalation system 100. In this sense, the tertiary indicator 364 has provided tactile feedback to the user at the second alert point, the second alert point corresponding to the point at which a cumulative volume of air flow through the air flow passage 322 has reached a predetermined volume of 15 litres since the indicator was last reset to zero. This may indicate that it is likely that the consumable of the inhaler article 200 has been fully consumed.

The inhalation volume estimator 350 further comprises a second stop 366. The second stop 366 is fixed to the housing 302 of the holder 300 and prevents anti-clockwise rotation of the indicator component 354 beyond the number 0 on the reference component. Thus, the stop and second stop 366 limit rotation of the indicator component 354 such that the indicator component 354 is always positioned between 0 and 15 on the reference component.

The holder 300 further comprises a modifying mechanism (not shown) coupled to the indicator for resetting or otherwise modifying the inhalation volume information.

In the embodiment of Figures 1 and 2, the modifying mechanism is operated by movement of the sleeve 306 downwards towards the piercing element 310. The mechanism is not shown in the Figures, but several options for this mechanism would be apparent to one skilled in the art after reading this disclosure. The modifying mechanism is configured such that downwards movement of the sleeve 306 resets the indicator component 354 so as to point at 0 on the reference component, and upwards movement of the sleeve 306 does not affect the indicator component 354.

The holder 300 shown in Figure 1 may additionally comprise an inhalation counter for counting a discrete number of inhalations on the inhalation system. The holder 300 may also comprise an inhalation count indicator for indicating information relating to the inhalation count to the user. The indicator and inhalation count indicator may both be simultaneously reset by a suitable modifying mechanism. The inhalation counter and inhalation count indicator are not shown in Figures 1 or 2.

As an example, the inhalation counter may comprise a pressure sensor in the air flow passage 322. The pressure sensor may be coupled to a battery of the holder. The inhalation count indicator may comprise an electronic display also coupled to the battery. As such, the inhalation counter may detect each inhalation, and increase an inhalation count shown on the display accordingly. A sensor could be used to detect motion of the sleeve 306 downwards to reset the inhalation count. Alternatively, a mechanical inhalation counter and mechanical inhalation count indicator may be used. In this case, there may be no need for a battery and the holder 300 may consist of non-electrical components.

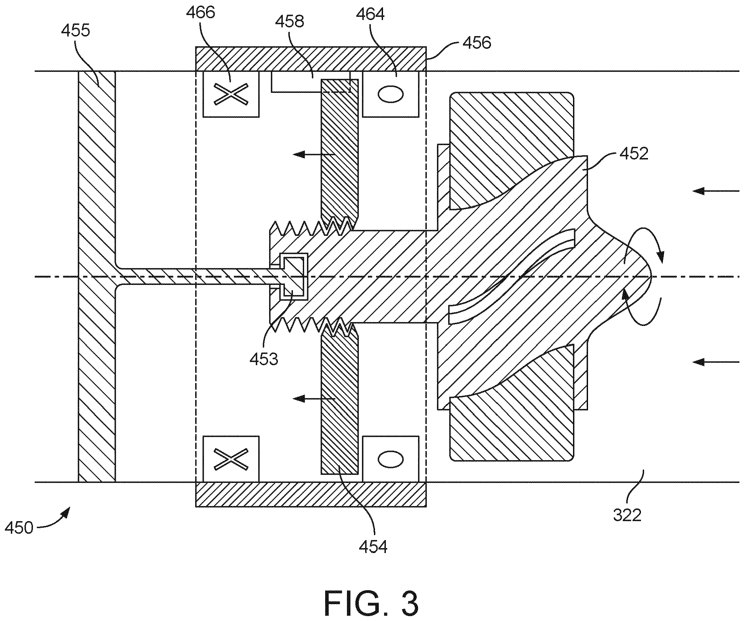

Figure 3 is a cross-sectional view of an alternative inhalation volume estimator 450. The alternative inhalation volume estimator 450 could replace the inhalation volume estimator 350 in the inhalation system 100 of Figure 1. The inhalation volume estimator 450 operates in a similar manner to the inhalation volume estimator 350 of Figure 2, and so only the differences between these two inhalation volume estimators are explained in detail here.

The inhalation volume estimator 450 of Figure 3 comprises an axial inflow turbine 452 and an indicator coupled to the turbine 452 for indicating inhalation volume information relating to a cumulative volume of all air flows through the air flow passage 322 since the indicator was last reset.

The turbine 452 is located in the air flow passage 322. The turbine 452 is coupled to the air flow passage 322 by a bearing 453 on a strut 455. The turbine 452 is therefore able to rotate relatively freely relative to the air flow passage 322 but cannot translate axially along the air flow passage 322.

The indicator comprises an indicator component 454. The indicator component 454 is substantially right cylindrical in shape and comprises an axially extending central aperture with an internal thread, and a plurality of axially extending holes located between the central hole and a substantially annular outer surface. The holes allow air to flow through the indicator component 454. In this sense, the indicator component 454 may be considered an air-permeable, threaded nut. The aperture with the internal thread is coupled to an external thread on a shaft of the turbine 452. A guide 458 located on an internal surface of the air flow passage 322 is engaged with an axially extending slot along the substantially annular outer surface of the indicator component 454 so as to prevent rotation of the indicator component 454.

The indicator also comprises a reference component 456. The reference component is in the form of a window on the housing of the holder. The indicator component 454 is visible through the reference component 456, or window, on the housing of the holder. The window does not extend around an entire circumference of the housing of the holder. Rather, the window is rectangular in shape and extends across a portion of a face of the housing of the holder.

In use, air flows through the air flow passage 322 as explained with reference to the inhalation system 100 of Figure 1. This air flow through the air flow passage 322 impinges upon the blades of the turbine 452 and causes the turbine 452 to rotate. The coupling between the cooperating threads of the indicator component 454 and the shaft of the turbine 452 convert this rotation of the turbine 452 into axial translation of the indicator component 454 relative to the reference component 456. This axial translation of the indicator component 454 relative to the reference component 456, or window, can be seen through the window and thus alters the inhalation volume information indicated by the indicator.

The inhalation volume estimator 450 comprises a first stop 464 and a second stop 466. The first stop 464 is configured to contact the indicator component 454 to prevent the indicator component 454 from translating axially upstream beyond a first position. The second stop 466 is configured to contact the indicator component 454 to prevent the indicator component 454 from translating axially downstream beyond a second position.

In the embodiment shown in Figure 3, the first stop 464 includes a marking "0", and the second stop 466 includes a marking "X". These markings are visible through the window. In other embodiments, these markings could read "0%" and "100%", or "start" and "finish".

In use, the indicator component 454 may initially be located adjacent to, or in contact with, the first stop 464, as shown in Figure 3. A user may then inhale on the inhalation system, causing an air flow through the air flow passage 322 in the direction indicated in Figure 3. This will cause the turbine 454 to rotate in the direction indicated in Figure 3. Due to the cooperating threads of the indicator component 454 and the shaft of the turbine 452, this rotation of the turbine 452 causes the indicator component 454 to translate axially in the direction indicated in Figure 3. The user is able to see this translation relative to the reference component 456 through the reference component 456, or window. With each inhalation, more air flows through the air flow passage 322 and the further rotation of the turbine 452 causes the indicator component 454 to translate further in the direction shown in Figure 3. Thus, the indicator indicates to the user an indication of a cumulative volume of air flow through the air flow passage 322 since the indicator was last reset. In this embodiment, resetting of the indicator may refer to translation of the indicator component 454 back to its initial position adjacent to the first stop 464.

Eventually, the indicator component 454 translates so far so as to abut the second stop 466. This may be considered a final position of the indicator component 454. The second stop 466 prevents the indicator component 454 from translating further in this downstream direction. As such, further rotation of the turbine 454 is also prevented. The distance of travel of the indicator component 454 from the initial position to the final position may correspond to a cumulative volume of air flow through the air flow passage 322 reaching a predetermined threshold volume, for example the volume at which a consumable is likely fully consumed. The indicator component 454 abutting the second stop 466 is visible through the reference component 456, or window. Thus, the indicator may indicate to the user that a consumable of the inhaler article 200 is likely fully consumed.

The inhalation volume estimator 450 comprises a modifying mechanism (not shown) coupled to the indicator for resetting or otherwise modifying the inhalation volume information. In this embodiment, the modifying mechanism comprises a button and a winding mechanism located on the housing of the holder. Specifically, the modifying mechanism is configured such that a user is able to press the button so as to engage the winding mechanism with the turbine 452, and is then able to wind, or rotate, the winding mechanism so as to rotate the turbine 454 in a direction opposite to the direction indicated in Figure 3. This rotation of the turbine 454 thus causes axial translation of the indicator component 454 in an upstream direction, opposite to the direction indicated in Figure 3. The user is thus able to translate the indicator component 454 back to the initial position, at which point the first stop 464 stops further translation of the indicator component 454 in this upstream direction. The user may then release the button so as to disengage the winding mechanism from the turbine 452.

Other features of the inhalation volume estimator 450 are similar or identical to the inhalation volume estimator 350 of Figure 2 so are not discussed here.

For the purpose of the present description and of the appended claims, except where otherwise indicated, all numbers expressing amounts, quantities, percentages, and so forth, are to be understood as being modified in all instances by the term "about". Also, all ranges include the maximum and minimum points disclosed and include any intermediate ranges therein, which may or may not be specifically enumerated herein. In this context, therefore, a number A is understood as A ± 10 of A. Within this context, a number A may be considered to include numerical values that are within general standard error for the measurement of the property that the number A modifies. The number A, in some instances as used in the appended claims, may deviate by the percentages enumerated above provided that the amount by which A deviates does not materially affect the basic and novel characteristic(s) of the claimed invention. Also, all ranges include the maximum and minimum points disclosed and include any intermediate ranges therein, which may or may not be specifically enumerated herein.

## Claims

1. A holder (300) for an inhaler article (200), the holder comprising an air flow passage (322), **characterised in that** the holder (300) comprises an inhalation volume estimator (350, 450), the inhalation volume estimator comprising:
a turbine (352, 452);
an indicator coupled to the turbine for indicating inhalation volume information relating to a volume of an air flow through the air flow passage; and
a modifying mechanism coupled to the indicator for resetting or otherwise modifying the inhalation volume information,
wherein the turbine is configured to rotate in response to air flow through the air flow passage and alter the inhalation volume information indicated by the indicator,
and wherein the modifying mechanism is operable by one or both of engaging the inhaler article with the holder and disengaging the inhaler article from the holder.

2. A holder (300) according to claim 1, wherein the indicator is coupled to the turbine (352, 452) so as to rotate when the turbine rotates.

3. A holder (300) according to claim 2, wherein a rate of rotation of the indicator is less than a rate of rotation of the turbine (352, 452).

4. A holder (300) according to any preceding claim, wherein the indicator is able to rotate indefinitely in a given direction.

5. A holder (300) according to any preceding claim, wherein the indicator comprises an indicator component (354, 454) coupled to the turbine (352, 452), and a reference component, and the indicator component is configured to move relative to the reference component as the turbine rotates.

6. A holder (300) according to any preceding claim, wherein the indicator is configured to indicate inhalation volume information relating to a cumulative volume of a plurality of temporally separated air flows through the air flow passage (322).

7. A holder (300) according to any preceding claim, wherein the inhalation volume estimator (350) comprises a secondary indicator (358) configured to alert the user at an alert point.

8. A holder (300) according to claim 7, wherein the secondary indicator (358) is configured to alert the user at the alert point with one or both of tactile and audible feedback.

9. A holder (300) according to claim 7 or 8, wherein the alert point corresponds to a cumulative volume of air flow through the air flow passage (322) reaching a predetermined volume.

10. A holder (300) according to any preceding claim, wherein the turbine is a radial inflow turbine (352).

11. A holder (300) according to any of claims 1 to 9, wherein the turbine is an axial inflow turbine (452).

12. A holder (300) according to any preceding claim, wherein the modifying mechanism is configured to allow the user to set the inhalation volume information.

13. A holder (300) according to any preceding claim wherein, in use, the holder is configured to interact with the inhaler article (200) so as to generate or introduce an aerosol downstream of the turbine.

14. A holder (300) according to any preceding claim, wherein the holder comprises an inhalation counter for counting a number of inhalations on the holder.

15. An inhalation system (100) comprising a holder (300) according to any preceding claim and an inhaler article (200) comprising a source of an aerosol component.

## Patentansprüche

1. Halter (300) für einen Inhalatorartikel (200), wobei der Halter einen Luftströmungskanal (322) umfasst, **dadurch gekennzeichnet, dass** der Halter (300) einen Inhalationsvolumenschätzer (350, 450) umfasst, wobei der Inhalationsvolumenschätzer umfasst:
eine Turbine (352, 452);
einen mit der Turbine gekoppelten Indikator zum Anzeigen von Inhalationsvolumeninformationen in Bezug auf ein Volumen einer Luftströmung durch den Luftströmungskanal; und
einen mit dem Indikator gekoppelten Änderungsmechanismus zum Zurücksetzen oder anderweitigen Ändern der Inhalationsvolumeninformationen,
wobei die Turbine zum Drehen in Reaktion auf eine Luftströmung durch den Luftströmungskanal und zum Ändern der durch den Indikator angezeigten Inhalationsvolumeninformationen ausgelegt ist,
und wobei der Änderungsmechanismus durch eines oder beides von Verbinden des Inhalatorartikels mit dem Halter und Trennen des Inhalatorartikels von dem Halter betätigt werden kann.

2. Halter (300) nach Anspruch 1, wobei der Indikator mit der Turbine (352, 452) gekoppelt ist, um sich bei Drehung der Turbine zu drehen.

3. Halter (300) nach Anspruch 2, wobei eine Geschwindigkeit der Drehung des Indikators geringer ist eine Geschwindigkeit der Drehung der Turbine (352, 452).

4. Halter (300) nach einem beliebigen vorhergehenden Anspruch, wobei sich der Indikator unendlich in eine bestimmte Richtung drehen kann.

5. Halter (300) nach einem beliebigen vorhergehenden Anspruch, wobei der Indikator eine mit der Turbine (352, 452) gekoppelte Indikatorkomponente (354, 454) und eine Referenzkomponente aufweist und die Indikatorkomponente zum Bewegen relativ zu der Referenzkomponente bei Drehung der Turbine ausgelegt ist.

6. Halter (300) nach einem beliebigen vorhergehenden Anspruch, wobei der Indikator zum Angeben von Inhalationsvolumeninformationen in Bezug auf ein kumulatives Volumen einer Vielzahl von zeitlich getrennten Luftströmungen durch den Luftströmungskanal (322) ausgelegt ist.

7. Halter (300) nach einem beliebigen vorhergehenden Anspruch, wobei der Inhalationsvolumenschätzer (350) einen Sekundärindikator (358) aufweist, der zum Warnen des Benutzers an einem Warnpunkt ausgelegt ist.

8. Halter (300) nach Anspruch 7, wobei der Sekundärindikator (358) zum Warnen des Benutzers an dem Warnpunkt mit einer oder beiden einer taktilen und akustischen Rückmeldung ausgelegt ist.

9. Halter (300) nach Anspruch 7 oder 8, wobei der Alarmpunkt einem kumulativen Volumen der Luftströmung durch den Luftströmungskanal (322) entspricht, das ein vorbestimmtes Volumen erreicht.

10. Halter (300) nach einem beliebigen vorhergehenden Anspruch, wobei die Turbine eine radiale Einströmungsturbine (352) ist.

11. Halter (300) nach einem beliebigen der Ansprüche 1 bis 9, wobei die Turbine eine axiale Einströmungsturbine (452) ist.

12. Halter (300) nach einem beliebigen vorhergehenden Anspruch, wobei der Änderungsmechanismus ausgelegt ist, dem Benutzer das Einstellen der Inhalationsvolumeninformationen zu ermöglichen.

13. Halter (300) nach einem beliebigen vorhergehenden Anspruch, wobei der Halter bei Gebrauch zum Zusammenwirken mit dem Inhalationsartikel (200) ausgelegt ist, um stromabwärts der Turbine ein Aerosol zu erzeugen oder einzuleiten.

14. Halter (300) nach einem beliebigen vorhergehenden Anspruch, wobei der Halter einen Inhalationszähler zum Zählen einer Anzahl von Inhalationen an dem Halter umfasst.

15. Inhalationssystem (100), umfassend einen Halter (300) nach einem beliebigen vorhergehenden Anspruch und einen Inhalatorartikel (200), umfassend eine Quelle einer Aerosolkomponente.

## Revendications

1. Support (300) pour un article inhalateur (200), le support comprenant un passage d'écoulement d'air (322), **caractérisé en ce que** le support (300) comprend un estimateur de volume d'inhalation (350, 450), l'estimateur de volume d'inhalation comprenant :
une turbine (352, 452) ;
un indicateur couplé à la turbine pour indiquer des informations de volume d'inhalation concernant un volume d'un écoulement d'air à travers le passage d'écoulement d'air ; et
un mécanisme de modification couplé à l'indicateur pour réinitialiser ou autrement modifier les informations de volume d'inhalation,
dans lequel la turbine est configurée pour tourner en réponse à un écoulement d'air à travers le passage d'écoulement d'air et modifier les informations de volume d'inhalation indiquées par l'indicateur,
et dans lequel le mécanisme de modification peut être mis en fonctionnement par l'un ou les deux parmi la mise en prise de l'article inhalateur avec le support et le dégagement de prise de l'article inhalateur avec le support.

2. Support (300) selon la revendication 1, dans lequel l'indicateur est couplé à la turbine (352, 452) de manière à tourner lorsque la turbine tourne.

3. Support (300) selon la revendication 2, dans lequel une vitesse de rotation de l'indicateur est inférieure à une vitesse de rotation de la turbine (352, 452).

4. Support (300) selon l'une quelconque des revendications précédentes, dans lequel l'indicateur est apte à tourner indéfiniment dans un sens donné.

5. Support (300) selon l'une quelconque des revendications précédentes, dans lequel l'indicateur comprend un composant indicateur (354, 454) couplé à la turbine (352, 452), et un composant de référence, et le composant indicateur est configuré pour se déplacer par rapport au composant de référence lorsque la turbine tourne.

6. Support (300) selon l'une quelconque des revendications précédentes, dans lequel l'indicateur est configuré pour indiquer des informations de volume d'inhalation concernant un volume cumulé d'une pluralité d'écoulements d'air séparés dans le temps à travers le passage d'écoulement d'air (322).

7. Support (300) selon l'une quelconque des revendications précédentes, dans lequel l'estimateur de volume d'inhalation (350) comprend un indicateur secondaire (358) configuré pour alerter l'utilisateur au niveau d'un point d'alerte.

8. Support (300) selon la revendication 7, dans lequel l'indicateur secondaire (358) est configuré pour alerter l'utilisateur au niveau du point d'alerte avec l'un ou les deux parmi une rétroaction tactile et audible.

9. Support (300) selon la revendication 7 ou 8, dans lequel le point d'alerte correspond à un volume cumulé d'écoulement d'air à travers le passage d'écoulement d'air (322) atteignant un volume prédéterminé.

10. Support (300) selon l'une quelconque des revendications précédentes, dans lequel la turbine est une turbine radiale (352) .

11. Support (300) selon l'une quelconque des revendications 1 à 9, dans lequel la turbine est une turbine axiale (452).

12. Support (300) selon l'une quelconque des revendications précédentes, dans lequel le mécanisme de modification est configuré pour permettre à l'utilisateur de définir les informations de volume d'inhalation.

13. Support (300) selon l'une quelconque des revendications précédentes, dans lequel, en utilisation, le support est configuré pour interagir avec l'article inhalateur (200) de manière à générer ou introduire un aérosol en aval de la turbine.

14. Support (300) selon l'une quelconque des revendications précédentes, dans lequel le support comprend un compteur d'inhalations pour compter un nombre d'inhalations sur le support.

15. Système d'inhalation (100) comprenant un support (300) selon l'une quelconque des revendications précédentes et un article inhalateur (200) comprenant une source d'un composant aérosol.
